# EUROPÄISCHE PATENTSCHRIFT

(11) **EP 1 082 088 B1**
(45) Veröffentlichungstag und Bekanntmachung des Hinweises auf die Patenterteilung: **10.09.2003**
(21) Anmeldenummer: 99926385.8
(22) Anmeldetag: 25.05.1999
(51) Int. Cl.: A61K 7/135

(54) **Verwendung eines Mittels zum Blondieren menschlicher Haare**
Use of a composition for bleaching human hair
Utilisation d'une composition pour blanchir des cheveux humains

(30) Priorität: 03.06.1998 DE 19824685
(43) Veröffentlichungstag der Anmeldung: 14.03.2001
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); NEUHAUS, Winifried, D-40822 Mettmann (DE)
(86) Internationale Anmeldenummer: EP9903564
(87) Internationale Veröffentlichungsnummer: WO99062469

(56) Entgegenhaltungen:
- EP-A- 0 168 719
- CH-A- 632 410
- DE-A- 19 548 132
- DE-A- 19 608 541
- FR-A- 2 126 256
- FR-A- 2 276 809
- FR-A- 2 317 910
- GB-A- 2 033 939
- US-A- 5 628 991

## Beschreibung

Die Erfindung betrifft die Verwendung eines Mittels zum Blondieren menschlicher Haare in Form eines Pulvers oder einer Paste.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und in einschlägigen Monographien. z.B. von K. Schrader, Grundlagen und Rezepturen der Kosmetika, 2. Auflage. 1989. Dr. Alfred Hüthig Verlag, Heidelberg, oder W. Umbach (Hrg.). Kosmetik, 2. Auflage, 1995, Georg Thieme Verlag, Stuttgart, New York, zusammenfassend beschrieben.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung üblicherweise mit einer Wasserstoffperoxidlösung vermischt werden, werden im weiteren als "Blondiermittel" bezeichnet. Alle aufgeführten Mengenangaben beziehen sich, soweit nicht anders ausgeführt, ausschließlich auf diese Zubereitungen.

Weder die pastenförmigen noch die pulverförmigen Blondiermittel, die heute auf dem Markt sind. können als optimal angesehen werden. Während die Blondierwirkung auf dem Haar als befriedigend bezeichnet werden kann, bestehen doch noch eine Reihe von Nachteilen und Problemen sowohl bei der Herstellung als auch bei der Handhabung dieser Mittel. Bei pastenförmigen Mitteln, die aus Stabilitätsgründer, hochviskos eingestellt werden, können insbesondere die Dosierung und das Mischungsverhalten in der Wasserstoffperoxidlösung noch nicht befriedigen. Ferner ist das Staubverhalten bei der Konfektionierung von Pasten ein Problem. Bei pulverförmigen Mitteln stehen das Staubverhalten, sowohl bei der Herstellung als auch bei der Anwendung, sowie ebenfalls das Mischungsverhalten bei der Anwendung im Mittelpunkt der Verbesserungsbemühungen.

In der EP-B1-0 560 088 wurde beispielsweise vorgeschlagen, das Staubverhalten von Blondierpulvern durch Zugabe von Ölen oder flüssigen Wachsen zu verbessern. In der deutscher Anmeldung DE-A1-196 00 216 war weiterhin vorgeschlagen worden, zur Entstaubung spezielle Ether in Mengen von 4 - 20 Gew.-% bezogen auf das gesamte Blondierpulver, einzusetzen. Pastenförmige Blondiermittel wurden in der DE-A1-38 14 356 zur Vermeidung von Staubbildung bei der Verarbeitung beschrieben. Dokument DE-A-19 548 132 beschreibt Mittel zum Blondieren menschlicher Haare auf Basis mindestens einer festen Peroxoverbindung und mindestens eines festen Alkaliträgers, die als Pulver vorliegen und 4-20 Gew.-% einer nichtionogenen grenzflächenaktiven Substanz enthalten.

Bei der Formulierung von Blondiermitteln muß ferner berücksichtigt werden, daß diese Mittel häufig Ammoniumsalze, wie z.B. Ammoniumchlorid oder Ammoniumperoxidisulfat enthalten. Basische Komponenten können während der Lagerung mit diesen Salzen unter Freisetzung von Ammoniak reagieren und somit zu einer Geruchsbelästigung führen bzw. die Lagerstabilität herabsetzen.

Es wurde nun überraschenderweise gefunden, dass Mittel, die bestimmte Substanzen aus der Gruppe der Amine und Ammoniumverbindungen enthalten und sich durch ein hervorragendes Staubverhalten, eine sehr guter Lagerfähigkeit und weitere vorteilhafte Eigenschaften auszeichnen, zur Blondierung menschlicher Haare verwendet werden können.

Gegenstand der vorliegenden Erfindung sind daher Mittel zum Blondieren menschlicher Haare auf Basis mindestens einer festen Peroxoverbindung und mindestens eines festen Alkaliträgers, dadurch gekennzeichnet, daß sie als Pulver oder als Paste vorliegen und 0,1 bis 25 Gew.-% einer Verbindung der Formeln (I) bis (III), 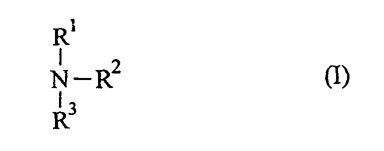 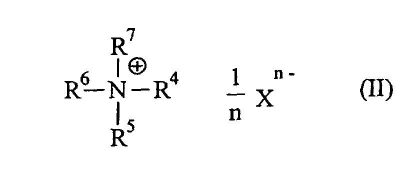 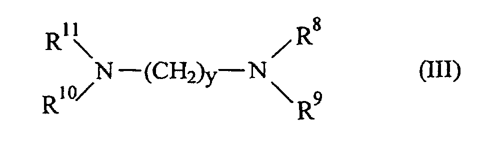 wobei die Reste R¹ bis R¹¹ unabhängig voneinander stehen fiir Wasserstoff, verzweigte oder unverzweigte C₁- bis C₂₂-Alkylgruppen, verzweigte oder unverzweigte, einfach oder mehrfach ungesättigte C₁- bis C₂₂-Alkylgruppen, verzweigte oder unverzweigte C₁- bis C₂₂-Hydroxyalkylgruppen oder Substituenten der Formeln (IVa), (IVb) oder (V) 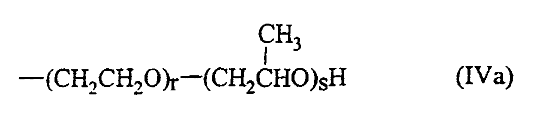 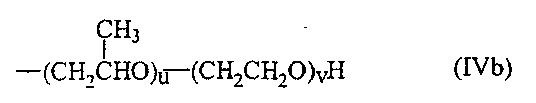 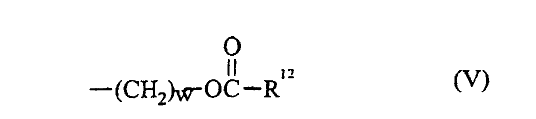 wobei r, s, u und v unabhängig voneinander von 0 bis 6 variieren können, w eine ganze Zahl von 1 bis 4 und R¹² eine verzweigte oder unverzweigte, gesättigte, einfach oder mehrfach ungesättigte C₁- bis C₂₂-Alkylgruppe sein kann, X ein Anion einer physiologisch verträglichen, anorganischen oder organischen Säure, n eine ganze Zahl und y eine ganze Zahl von 1 bis 6 ist, mit den Maßgaben, daß bei Verbindungen der Formel (I) mindestens einer der Reste R¹ bis R³ eine C₁- bis C₂₂-Kette enthalten muß, bei Verbindungen der Formel (II) mindestens einer der Reste R⁴ bis R⁷ ausgewählt ist aus Substituenten der Formeln (IVa) oder (IVb), und der Schmelzpunkt der Verbindungen der Formel (I) bis (III) unterhalb von 60 °C liegt, enthalten

Die erfindungsgemäß verwendeten Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den Blondiermitteln bevorzugt in Mengen von 20-80 Gew.-%, insbesondere in Mengen von 40-70 Gew.-%, enthalten.

Als weitere zwingende Komponente enthalten die erfindungsgemäß verwendeten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Die Blondiermittel enthalten Alkalisierungsmittel bevorzugt in Mengen von 10-30 Gew.-%, insbesondere 15-25 Gew.-%.

Erfindungswesentlich ist die dritte zwingende Komponente der Blondiermittel, die Verbindung gemäß einer der Formeln (I) bis (III).

Als besonders geeignet haben sich Verbindungen gemäß Formel (I) bis (III) erwiesen, bei denen einer der Substituenten eine verzweigte oder unverzweigte, gesättigte, einfach oder mehrfach ungesättigte C₁- bis C₂₂-Alkylgruppe ist. Dabei kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält. Solche Verbindungen werden im Folgenden z.B. als Kokosamin, Talgamin oder Sojaamin bezeichnet.

Beispiele für Amine gemäß Formel (I) sind das Monoethanolamin, Triethanolamin sowie das 2-Amino-2-methyl-1-propanol.

Besonders bevorzugte Amine gemäß Formel (I) sind ethoxylierte Kokosamine und Derivate des Sojaamins, insbesondere das PEG-3-Cocamine und das Dihydroxyethyl Soyamine Dioleate.

Die Verbindungen der Formel (II) liegen erfindungsgemäß als physiologisch verträgliche Salze mit anorganischen oder organischen Säuren vor. Beispiele für solche Salze sind die Chloride, die Bromide, die Sulfate, die Phosphate, die Acetate, die Propionate. die Citrate und die Lactate. Erfindungsgemäß bevorzugt sind die Chloride. Beispiele für Verbindungen der Formel (II) sind die unter dem Handelsnamen Ethoquad® vertriebenen ethoxylierten quartären Ammoniumverbindungen der Firma Akzo Nobel.

Eine besonders bevorzugte Verbindung der Formel (III) ist das unter dem Handelsnamen Ethoduomeen® T20 vertriebene PEG-10 Tallow Aminopropylamine, als Beispiel für ein ethoxyliertes Derivat des Talgamins.

Die Blondiermittel enthalten die Verbindungen der Formeln (I) bis (III) bevorzugt in einer Menge von 0,8-25 Gew.-%, ganz besonders bevorzugt in einer Menge von 0.8-12 Gew.-%, insbesondere in einer Menge von 1-8 Gew.-%.

Weiterhin hat es sich als vorteilhaft erwiesen, wenn die Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeilbarkeit Substanzen, die kommerziell als Feststoffe oder Flüssigkeiten in reiner Form erhältlich sind. Die Definition fiir Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere I bis 15, Ethylenoxidund/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C₁-C₄-Alkyl-Ethers, "verschlossen". Ein Beispiel für eine solche Verbindung ist das Handelsprodukt Dehypon®LT 054, ein C₁₂₋₁₈-Fettalkoholol + 4,5 Ethylenoxidbutylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxystearat (Handelsprodukt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol - 4 EO, Nonylphenol + 9 EO, Octylphenol + 3 EO und Octylphenol - 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich Mittel erwiesen, die nichtionogene grenzflächenaktive Substanzen in Mengen von 0,5 - 10 Gew.-% enthalten.

Weiterhin können die Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C₈-C₂₂-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C₈-C₂₂-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid S 18 erhältliche Stearylamidopropyldimethylamin, zeichnen sich neben einer guter, konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohol erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-. Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel 1 wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine,
- Fettalkohole und Fettsäureester,
- Fettsäurealkanolamide,
- Komplexbildner wie EDTA, NTA und Phosphonsäuren,
- Quell- und Penetrationshilfsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

In einer bevorzugten Ausführungsform, insbesondere wenn das Haar nicht übermäßig beschwert werden soll, sind die Mittel im übrigen frei von Ölen und flüssigen Wachsen. Dabei ist klarzustellen, daß der Begriff Öle die bekannten fetten und synthetischen Öle, nicht aber Parfümöle umfaßt, die selbstverständlich in geringen Mengen als Duftstoffe eingesetzt werden können.

Die Herstellung der erfindungsgemäß verwendteten Blondiermittel kann nach den üblichen, dem Fachmann bekannten Verfahren erfolgen.

Ein bevorzugtes Verfahren besteht darin, die anorganischen, als Feststoff vorliegenden Komponenten, gegebenenfalls nach Mischung z.B. in einem Drais-Mischer, vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die Blondiermittel ist das Vermahlen aller Komponenten in einer Kugelmühle, einer Ringwalzenmühle oder insbesondere einer Spindelmühle.

Schließlich ist es möglich, die pulverförmigen Blondiermittel durch Mischen aller Komponenten und die anschließende Behandlung, bevorzugt bei erhöhten Temperaturen, im Wirbelbett herzustellen.

Für die Anwendung der Mittel auf dem Haar werden die Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Blondiermittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

### Beispiele

Es wurden 11 Blondiermittel zubereitet, deren Zusammensetzung in Gew.-% in Tabelle 1 angegeben sind. In den Beispielen A1 bis A5 wurden verschiedene Blondiermittel, die frei von Ammoniumsalzen sind, mit primären Aminen entstaubt. In den Beispielen B1 bis B5 wurde das Verhalten von Blondiermitteln mit einem ethoxylierten Kokosamin untersucht. Während Beispiel B1 frei von Ammoniumsalzen ist, enthalten die Beipiele B2 bis B5 übliche Ammoniumsalze. Das Beispiel V ist ein Vergleichszubereitung ohne Ammoniumsalze und frei von Verbindungen der Formeln (I) bis (III).

Alle Mittel außer der Vergleichszubereitung V waren staubfrei.

Alle Mittel ergaben beim Vermischen mit einem 6%igen Entwickler (Marktprodukt Poly Brillance) im Verhältnis 1:1 innerhalb kurzer Zeit homogene Mischungen (Vergleichzubereitung V wurde im Verhältnis 0,92:1 gemischt).

Die ammoniumsalzhaltigen Mischungen (B2-B5) zeigten unmittelbar nach der Herstellung und auch nach 4-monatiger Lagerung bei Raumtemperatur keinen Ammoniakgeruch.

Dunkelblonde Haarsträhnen (Fischbach + Miller, Code 6923) von ca. 0,5g Gewicht wurden mit jeweils ca. 2g Blondiermittel 30min lang blondiert. Im Anschluß erfolgte eine visuelle Beurteilung im Vergleich zu Rezepturen, die keine Entstaubungsmittel enthielten. Eine Beeinträchtigung der Aufhellwirkung der erfindungsgemäß verwendeten Mittel konnte nicht festgestellt werden.

**Tabelle 1:**

| Komponenten | A1 | A2 | A3 | A4 | A5 | B1 | B2 | B3 | B4 | B5 | V |
|---|---|---|---|---|---|---|---|---|---|---|---|
| Ammoniumperoxidisulfat | - | - | - | - | - | - | 19,0 | 19,0 | - | - | - |
| Kaliumperoxidisulfat | 55,5 | 53,5 | 51,5 | - | - | 53,0 | 36,0 | - | 48,0 | - | 57,4 |
| Natriumperoxidisulfat | - | - | - | 56,5 | 46,5 | - | - | 36,0 | - | 45,0 | - |
| Natriummetasilikat | 18,0 | 18,0 | 18,0 | 18,0 | 18,0 | 18,0 | 18,0 | 18,0 | 18,0 | 18,0 | 19,4 |
| Magnesiumhydroxycarbonat | 3,0 | 3,0 | 3,0 | 3,0 | 3,0 | 3,0 | 3,0 | 3,0 | 3,0 | 3,0 | 3,2 |
| Natriumchlorid | - | 2,0 | 5,0 | - | - | 2,0 | - | - | 5,0 | - | 2,2 |
| Kaliumsulfat | - | - | - | - | 10,0 | - | - | - | - | 5,0 | - |
| Ammoniumsulfat | - | - | - | - | - | - | - | - | - | 5,0 | - |
| Ammoniumchlorid | - | - | - | - | - | - | - | - | 2,0 | - | - |
| Natriumstearat | 11,0 | 11,0 | 11,0 | 11,0 | 11,0 | 11,0 | 11,0 | 11,0 | 11,0 | 11,0 | 11,8 |
| Pyrogenes Siliziumdioxid | 2,5 | 2,5 | 2,5 | 2,5 | 2,5 | 2,5 | 2,5 | 2,5 | 2,5 | 2,5 | 2,7 |
| Dinatriumdihydrogenethylendiamintetraacetat | 1,0 | 1,0 | 1,0 | 1,0 | 1,0 | 1,0 | 1,0 | 1,0 | 1,0 | 1,0 | 1,1 |
| Monoethanolamin | 7,0 | 7,0 | - | - | - | - | - | - | - | - | - |
| 2-Amino-2-methyl-1-propanol | - | - | 8,0 | 8,0 | 8,0 | - | - | - | - | - | - |
| Dehydat®-50¹ | - | - | - | - | - | 7,5 | 7,5 | 7,5 | 7,5 | 7,5 | - |
| Trinatriumphosphat | 2,0 | 2,0 | - | - | - | 2,0 | 2,0 | 2,0 | 2,0 | 2,0 | 2,2 |
| Gesamtmenge | 100 | 100 | 100 | 100 | 100 | 100 | 100 | 100 | 100 | 100 | 100 |

| | | | | | | | | | | | |
|---|---|---|---|---|---|---|---|---|---|---|---|
| ¹ Bis (2-hydroxyethyl)kokosamin (HENKEL) | | | | | | | | | | | |

## Patentansprüche

1. Verwendung eines Mittel auf Basis mindestens einer festen Peroxoverbindung und mindestens eines festen Alkaliträgers zum Blondieren menschlicher Haare, **dadurch gekennzeichnet, dass** das Mittel als Pulver oder Paste vorliegt und 0,1 - 25 Gew.-% mindestens einer Verbindung der Formeln (I) bis (III) 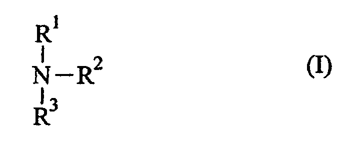 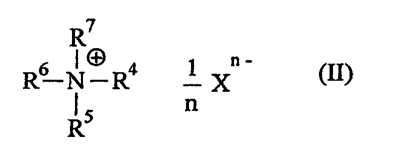 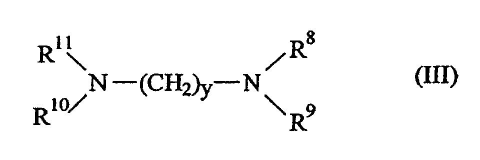 wobei die Reste R¹ bis R¹ unabhängig voneinander stehen für
- Wasserstoff
- verzweigte oder unverzweigte C₁- bis C₂₂-Alkylgruppen
- verzweigte oder unverzweigte, einfach oder mehrfach ungesättigte C₁bis C₂₂- Alkylgruppen
- verzweigte oder unverzweigte C₁- bis C₂₂-Hydroxyalkylgruppen
- oder Substituenten der Formeln (IVa), (IVb) bzw. (V) 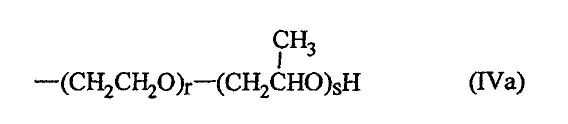 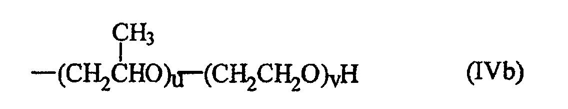 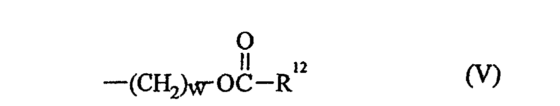 wobei r, s, u und v unabhängig voneinander von 0 bis 6 variieren können, w eine ganze Zahl von 1 bis 4 und R¹² eine verzweigte oder unverzweigte, gesättigte, einfach oder mehrfach ungesättigte C₁- bis C₂₂-Alkylgruppe sein kann,
X ein Anion einer physiologisch verträglichen, anorganischen oder organischen Säure, n eine ganze Zahl und y eine ganze Zahl von 1 bis 6 ist mit den Maßgaben, dass
- bei Verbindungen der Formel (I) mindestens einer der Reste R¹ bis R³ eine C₁- bis C₂₂-Kette enthalten muss,
- bei Verbindungen der Formel (II) mindestens einer der Reste R⁴ bis R⁷ ausgewählt ist aus Substituenten der Formeln (IVa) oder (IVb), und
- der Schmelzpunkt der Verbindungen der Formel (I) bis (III) unterhalb von 60 °C liegt,
enthält.

2. Verwendung nach Anspruch 1, **dadurch gekennzeichnet, dass** das Mittel eine anorganische feste Peroxoverbindung enthält.

3. Verwendung nach einem der Ansprüche 1 bis 2, **dadurch gekennzeichnet, dass** das Mittel eine Verbindung der Formel (I) enthält, mit der Maßgabe, dass mindestens einer der Substituenten eine verzweigte oder unverzweigte, gesättigte, einfach oder mehrfach ungesättigte C₁-bis C₂₂-Alkylgruppe ist.

4. Verwendung nach einem der Ansprüche 1 bis 3, **dadurch gekennzeichnet, dass** das Mittel als Verbindung der Formel (I) ein Derivat des Kokosamins enthält, insbesondere eine ethoxyliertes Kokosamin.

5. Verwendung nach einem der Ansprüche 1 bis 4, **dadurch gekennzeichnet, dass** das Mittel als Verbindung der Formel (I) ein Derivat des Sojaamins enthält.

6. Verwendung nach einem der Ansprüche I bis 5, **dadurch gekennzeichnet, dass** das Mittel als Verbindung der Formel (III) ein ethoxyliertes Derivat des Talgamins enthält.

7. Verwendung nach einem der Ansprüche 1 bis 6, **dadurch gekennzeichnet, dass** die Verbindungen der Formeln (I) bis (III) in einer Menge von 0,8 - 12 Gew.-%, insbesondere in einer Menge von 1 bis 8 Gew.-%, enthalten sind.

8. Verwendung nach einem der Ansprüche 1 bis 7, **dadurch gekennzeichnet, dass** das Mittel nichtionogene grenzflächenaktive Substanzen in Mengen von 0,5 - 10 Gew.-% enthält.

9. Verwendung einem der Ansprüche 1 bis 8, **dadurch gekennzeichnet, dass** das Mittel eine nichtionogene grenzflächenaktive Substanz, ausgewählt aus
- alkoxylierten Fettalkoholen und Fettsäuren mit 8 bis 22 Kohlenstoffatomen und 1 bis 30 Ethylenoxid- und/oder Propylenoxid-Einheiten, die eine Alkylendgruppe, insbesondere eine Methylgruppe, am Ende der Alkoxygruppenkette aufweisen können,
- alkoxylierten Mono-, Di- und Triglyceriden,
- Polyglycerinestern und alkoxylierten Polyglycerinestern,
- Sorbitan-Fettsäureestem und alkoxylierten Sorbitan-Festtsäureestern,
- Alkylphenolalkoxylaten mit 6 bis 21 Kohlenstoffatomen in der Alkylkette und 0 bis 30 Ethylenoxid- und/oder Propylenoxid-Einheiten
enthält.

10. Verwendung nach einem der Ansprüche 1 bis 9, **dadurch gekennzeichnet, dass** das Mittel bei Raumtemperatur feste Seifen, insbesondere Natriumstearat, in Mengen von 5-20 Gew.-%, insbesondere 10-15 Gew.-%, bezogen auf das gesamte Blondiermittel, enthält.

11. Verwendung nach einem der Ansprüche 1 bis 10, **dadurch gekennzeichnet, dass** das Mittel im übrigen frei von Ölen und flüssigen Wachsen ist, wobei Parfümöle ausgenommen sind.

12. Mittel zum Blondieren menschlicher Haare auf Basis mindestens einer festen Peroxoverbindung und mindestens eines festen Alkaliträgers, **dadurch gekennzeichnet, dass** es als Pulver oder Paste vorliegt und 0,1 - 25 Gew.-% mindestens einer Verbindung der Formeln (I) bis (III) 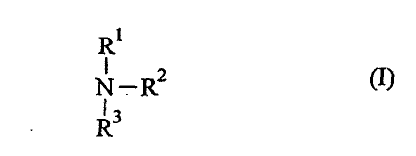 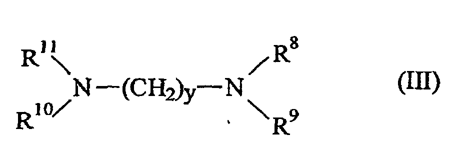 wobei die Reste R¹ bis R³ und R⁸ bis R¹¹ unabhängig voneinander stehen für
- Wasserstoff
- verzweigte oder unverzweigte C₁- bis C₂₂-Alkylgruppen
- verzweigte oder unverzweigte, einfach oder mehrfach ungesättigte C₁bis C₂₂- Alkylgruppen
- verzweigte oder unverzweigte C₁- bis C₂₂-Hydroxyalkylgruppen
- oder Substituenten der Formeln (IVa), (IVb) bzw. (V) 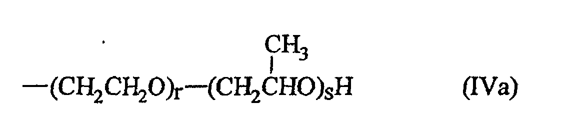 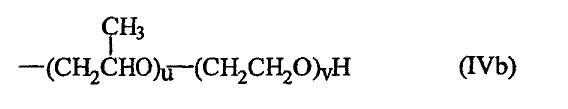 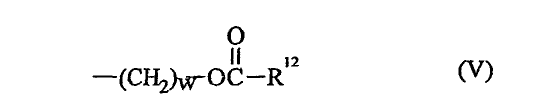 wobei r, s, u und v unabhängig voneinander von 0 bis 6 variieren können, w eine ganze Zahl von 1 bis 4 und R¹² eine verzweigte oder unverzweigte, gesättigte, einfach oder mehrfach ungesättigte C₁- bis C₂₂-Alkylgruppe sein kann,
y eine ganze Zahl von 1 bis 6 ist mit den Maßgaben, dass
- bei Verbindungen der Formel (I) mindestens einer der Reste R¹ bis R³ eine C₁- bis C₂₂-Kette enthalten muss,
- der Schmelzpunkt der Verbindungen der Formel (I) und (III) unterhalb von 60 °C liegt,
enthält.

13. Mittel zum Blondieren menschlicher Haare, erhältlich durch Mischung einer Wasserstoffperoxidlösung und eines Blondiermittels auf Basis mindestens einer festen Peroxoverbindung und mindestens eines festen Alkaliträgers, **dadurch gekennzeichnet, dass** das Blondiermittel als Pulver oder Paste vorliegt und 0,1 - 25 Gew.-% mindestens einer Verbindung der Formeln (I) bis (III) 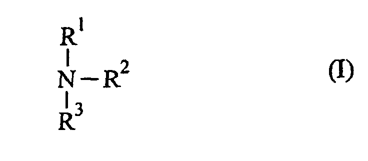 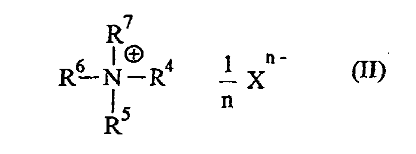 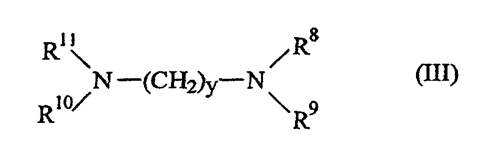 wobei die Reste R¹ bis R¹¹ unabhängig voneinander stehen für
- Wasserstoff
- verzweigte oder unverzweigte C₁- bis C₂₂-Alkylgruppen
- verzweigte oder unverzweigte, einfach oder mehrfach ungesättigte C₁bis C₂₂- Alkylgruppen
- verzweigte oder unverzweigte C₁- bis C₂₂-Hydroxyalkylgruppen
- oder Substituenten der Formeln (IVa), (IVb) bzw. (V) 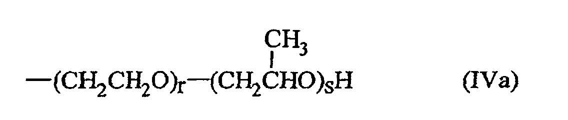 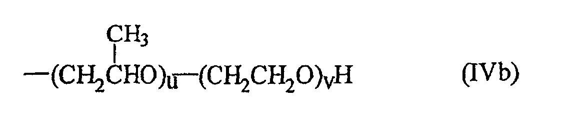 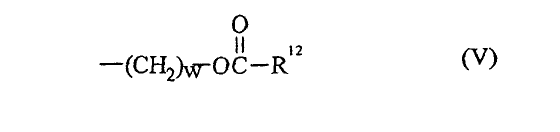 wobei r, s, u und v unabhängig voneinander von 0 bis 6 variieren können, w eine ganze Zahl von 1 bis 4 und R¹² eine verzweigte oder unverzweigte, gesättigte, einfach oder mehrfach ungesättigte C₁- bis C₂₂-Alkylgruppe sein kann,
X ein Anion einer physiologisch verträglichen, anorganischen oder organischen Säure, n eine ganze Zahl und y eine ganze Zahl von 1 bis 6 ist mit den Maßgaben, dass
- bei Verbindungen der Formel (I) mindestens einer der Reste R¹ bis R³ eine C₁- bis C₂₂-Kette enthalten muss,
- bei Verbindungen der Formel (II) mindestens einer der Reste R⁴ bis R⁷ ausgewählt ist aus Substituenten der Formeln (IVa) oder (IVb), und
- der Schmelzpunkt der Verbindungen der Formel (I) bis (III) unterhalb von 60 °C liegt,
enthält.

## Claims

1. Use of a composition based on at least one solid peroxo compound and at least one solid alkali carrier for bleaching human hair, **characterized in that** the composition is in the form of a powder or paste and comprises 0.1-25% by weight of at least one compound of the formulae (I) to (III) 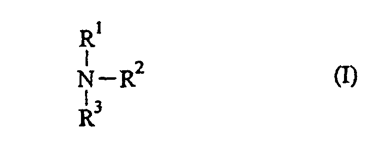 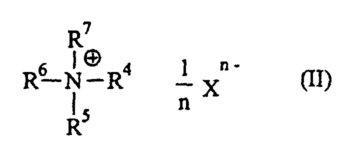 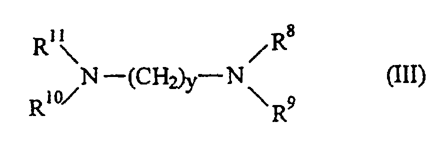 where the radicals R¹ to R¹¹, independently of one another, are
- hydrogen
- branched or unbranched C₁- to C₂₂-alkyl groups
- branched or unbranched, mono- or polyunsaturated C₁- to C₂₂-alkyl groups
- branched or unbranched C₁- to C₂₂-hydroxyalkyl groups
- or substituents of the formulae (IVa), (IVb) or (V) 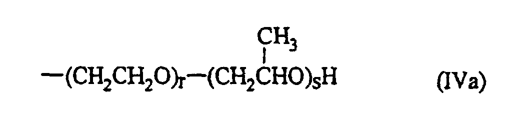 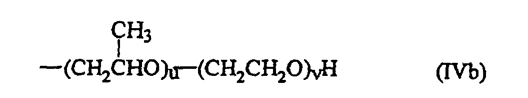 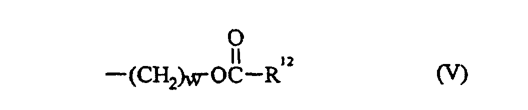 where r, s, u and v, independently of one another, can vary from 0 to 6, w may be an integer from 1 to 4 and R¹² may be a branched or unbranched, saturated, mono- or polyunsaturated C₁- to C₂₂-alkyl group,
X is an anion of a physiologically compatible inorganic or organic acid, n is an integer and y is an integer from 1 to 6 with the provisos that
- for compounds of the formula (I) at least one of the radicals R¹ to R³ must contain a C₁ to C₂₂ chain,
- for compounds of the formula (II) at least one of the radicals R⁴ to R⁷ is chosen from substituents of the formulae (IVa) or (IVb), and
- the melting point of the compounds of the formula (I) to (III) is below 60°C.

2. Use according to Claim 1, **characterized in that** the composition comprises an inorganic solid peroxo compound.

3. Use according to one of Claims 1 to 2, **characterized in that** the composition comprises a compound of the formula (I) with the proviso that at least one of the substituents is a branched or unbranched, saturated, mono- or polyunsaturated C₁- to C₂₂-alkyl group.

4. Use according to one of Claims 1 to 3, **characterized in that** the composition comprises a derivative of coconut amine, in particular an ethoxylated coconut amine, as compound of the formula (I).

5. Use according to one of Claims 1 to. 4, **characterized in that** the composition comprises a derivative of soya amine as compound of the formula (I).

6. Use according to one of Claims 1 to 5, **characterized in that** the composition comprises an ethoxylated derivative of tallow amine as compound of the formula (III).

7. Use according to one of Claims 1 to 6, **characterized in that** the compounds of the formulae (I) to (III) are present in an amount of 0.8-12% by weight, in particular in an amount of 1 to 8% by weight.

8. Use according to one of Claims 1 to 7, **characterized in that** the composition comprises nonionogenic interface-active substances in amounts of 0.5-10% by weight.

9. Use according to one of Claims 1 to 8, **characterized in that** the composition comprises a nonionogenic interface-active substance chosen from
- alkoxylated fatty alcohols and fatty acids having 8 to 22 carbon atoms and 1 to 30 ethylene oxide and/or propylene oxide units which can have an alkyl end group, in particular a methyl group, at the end of the alkoxy group chain,
- alkoxylated mono-, di- and triglycerides,
- polyglycerol esters and alkoxylated polyglycerol esters,
- sorbitan fatty acid esters and alkoxylated sorbitan fatty acid esters,
- alkylphenol alkoxylates having 6 to 21 carbon atoms in the alkyl chain and 0 to 30 ethylene oxide and/or propylene oxide units.

10. Use according to one of Claims 1 to 9, **characterized in that** the composition comprises soaps which are solid at room temperature, in particular sodium stearate, in amounts of 5-20% by weight, in particular 10-15% by weight, based on the total bleaching composition.

11. Use according to one of Claims 1 to 10, **characterized in that** the composition is otherwise free from oils and liquid waxes, with the exception of perfume oils.

12. Composition for bleaching human hair based on at least one solid peroxo compound and at least one solid alkali carrier, **characterized in that** it is in the form of a powder or paste and comprises 0.1-25% by weight of at least one compound of the formulae (I) and (III) 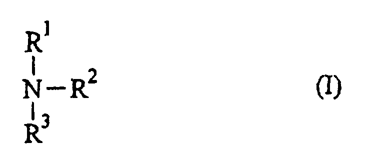 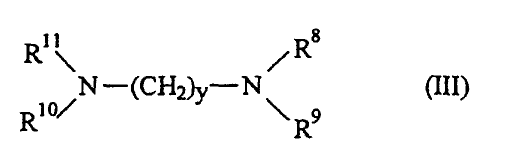 where the radicals R¹ to R³ and R⁸ to R¹¹, independently of one another, are
- hydrogen
- branched or unbranched C₁- to C₂₂-alkyl groups
- branched or unbranched, mono- or polyunsaturated C₁- to C₂₂-alkyl groups
- branched or unbranched C₁- to C₂₂-hydroxyalkyl groups
- or substituents of the formulae (IVa), (IVb) or (V) 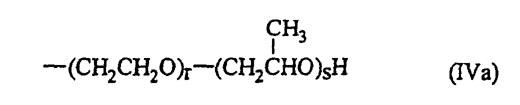 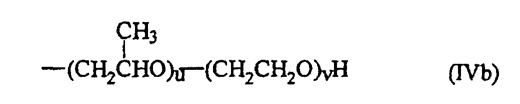 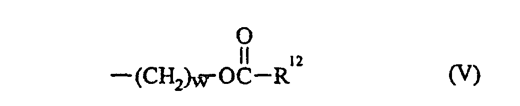 where r, s, u and v, independently of one another, can vary from 0 to 6, w may be an integer from 1 to 4 and R¹² may be a branched or unbranched, saturated, mono- or polyunsaturated C₁- to C₂₂-alkyl group,
y is an integer from 1 to 6, with the provisos that
- for compounds of the formula (I) at least one of the radicals R¹ to R³ must contain a C₁ to C₂₂ chain,
- the melting point of the compounds of the formula (I) to (III) is below 60°C.

13. Composition for bleaching human hair, obtainable by mixing a hydrogen peroxide solution and a bleaching composition based on at least one solid peroxide compound and at least one solid alkali carrier, **characterized in that** the bleaching composition is in the form of a powder or paste and comprises 0.1-25% by weight of at least one compound of the formulae (I) to (III) 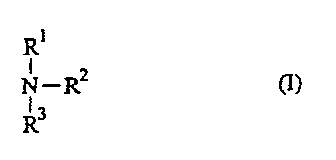 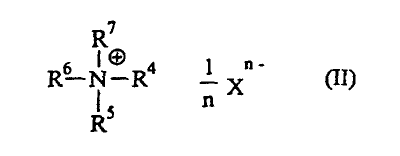 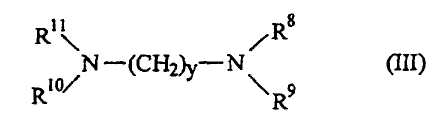 where the radicals R¹ to R¹¹, independently of one another, are
- hydrogen
- branched or unbranched C₁- to C₂₂-alkyl groups
- branched or unbranched, mono- or polyunsaturated C₁- to C₂₂-alkyl groups
- branched or unbranched C₁- to C₂₂-hydroxyalkyl groups
- or substituents of the formulae (IVa), (IVb) or (V) 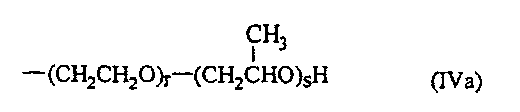 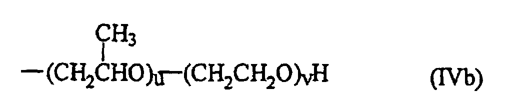 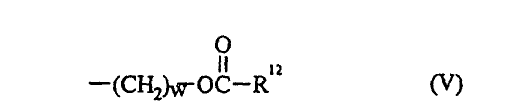 where r, s, u and v, independently of one another, can vary from 0 to 6, w may be an integer from 1 to 4 and R¹² may be a branched or unbranched, saturated, mono- or polyunsaturated C₁- to C₂₂-alkyl group,
X is an anion of a physiologically compatible inorganic or organic acid, n is an integer and y is an integer from 1 to 6 with the provisos that
- for compounds of the formula (I) at least one of the radicals R¹ to R³ must contain a C₁ to C₂₂ chain,
- for compounds of the formula (II) at least one of the radicals R⁴ to R⁷ is chosen from substituents of the formulae (IVa) or (IVb), and
- the melting point of the compounds of the formula (I) to (III) is below 60°C.

## Revendications

1. Utilisation d'un agent à base d'au moins un composé peroxo solide et d'au moins un véhicule solide d'alcali pour blondir les cheveux humains, **caractérisée en ce que** l'agent existe sous forme de poudre ou de pâte et contient de 0,1 à 25% en poids d'au moins un composé de formules (I) à (III) 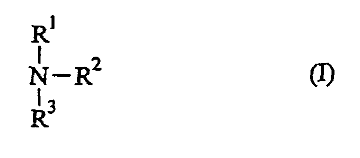 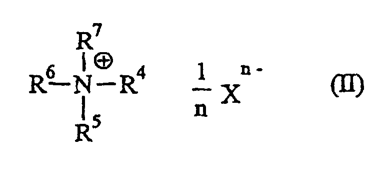 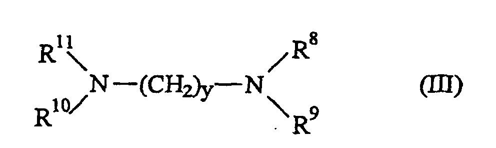 dans lesquelles les résidus R¹ à R¹¹ représentent indépendamment l'un de l'autre
- un atome d'hydrogène
- des groupes alkyle en C₁ à C₂₂, ramifiés ou non ramifiés
- des groupes alkyle en C₁ à C₂₂, mono- ou poly-insaturés, ramifiés ou non ramifiés
- des groupes hydroxyalkyle en C₁ à C₂₂, ramifiés ou non ramifiés
- ou des substituants de formules (IVa), (IVb), ou (V) 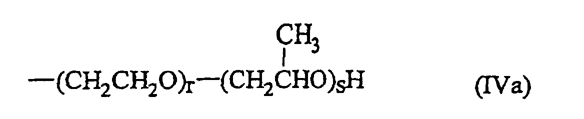 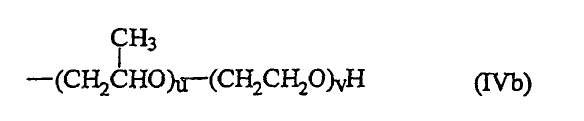 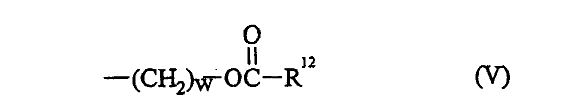 dans lesquelles r, s, u et v peuvent varier indépendamment l'un de l'autre de 0 à 6, w est un nombre entier de 1 à 4, et R¹² peut être un groupe alkyle en C₁ à C₂₂ mono- ou poly-insaturé, saturé, ramifié ou non ramifié,
X est un anion d'un acide inorganique ou organique, acceptable sur le plan physiologique, n est un nombre entier et y est un nombre entier de 1 à 6, avec les conditions :
- au moins un des résidus R¹ à R³ dans les composés de formule (I) doit contenir une chaîne en C₁ à C₂₂,
- au moins un des résidus R⁴ à R⁷ dans les composés de formule (II), est choisi parmi les substituants de formules (IVa) ou (IVb), et
- le point de fusion des composés de formule (I) à (III) est inférieur à 60°C.

2. Utilisation selon la revendication 1, **caractérisé en ce que** l'agent contient un composé peroxo solide inorganique.

3. Utilisation selon l'une quelconque des revendications 1 à 2, **caractérisée en ce que** l'agent contient un composé de formule (I), à condition qu'au moins un des substituants soit un groupe alkyle en C₁ à C₂₂, mono- ou poly-insaturé, saturé, ramifié ou non ramifié.

4. Utilisation selon l'une quelconque des revendications 1 à 3, **caractérisée en ce que** l'agent contient en tant que composé de formule (I), un dérivé de l'amine de coprah, en particulier une amine de coprah étoxylée.

5. Utilisation selon l'une quelconque des revendications 1 à 4, **caractérisée en ce que** l'agent en tant que composé de formule (I), contient un dérivé de l'amine de soja.

6. Utilisation selon l'une quelconque des revendications 1 à 5, **caractérisée en ce que** l'agent en tant que composé de formule (III), contient un dérivé éthoxylé de l'amine de suif.

7. Utilisation selon l'une quelconque des revendications 1 à 6, **caractérisée en ce que** les composés des formules (I) à (III), sont contenus en une quantité de 0,8 à 12% en poids, en particulier en une quantité de 1 à 8% en poids.

8. Utilisation selon l'une quelconque des revendications 1 à 7, **caractérisée en ce que** l'agent contient des substances tensio-actives non ionogènes en des quantités de 0,5 à 10% en poids.

9. Utilisation selon l'une quelconque des revendications 1 à 8, **caractérisée en ce que** l'agent contient une substance tensio-active non ionogène choisie parmi
- les alcools gras et acides gras alcoxylés ayant 8 à 22 atomes de carbone et 1 à 30 motifs oxyde d'éthylène et/ou oxyde de propylène, qui peuvent présenter un groupe terminal alkyle, en particulier un groupe méthyle, à l'extrémité de la chaîne de groupes alcoxy,
- les mono-, di- et tri-glycérides alcoxylés,
- les polyglycérine-esters et les polyglycérine-esters alcoxylés,
- les esters d'acide gras et de sorbitan, et les esters d'acide gras et de sorbitan alcoxylés,
- les alcoxylates d'alkylphénol ayant 6 à 21 atomes de carbone dans la chaîne alkyle, et 0 à 30 motifs oxyde d'éthylène et/ou oxyde de propylène.

10. Utilisation selon l'une quelconque des revendications 1 à 9, **caractérisée en ce que** l'agent contient des savons solides à la température ambiante, en particulier le stéarate de sodium, en des quantités de 5 à 20% en poids, en particulier de 10 à 15% en poids, par rapport à l'agent blondissant total.

11. Utilisation selon l'une quelconque des revendications 1 à 10, **caractérisée en ce que** l'agent est par ailleurs exempt d'huiles et de cires liquides, les huiles de parfum étant exclues.

12. Agent pour blondir les cheveux humains à base d'au moins un composé peroxo solide, et d'au moins un véhicule solide d'alcali, **caractérisé en ce qu'**il existe sous forme de poudre ou de pâte, et contient 0,1 à 25% en poids d'au moins un composé des formules (I) à (III) 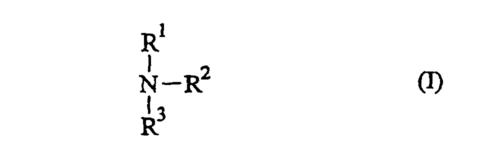 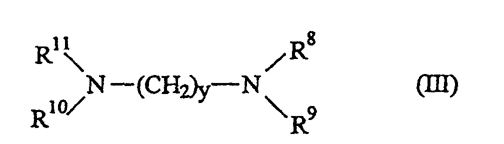 dans lesquelles les résidus R¹ à R³ et R⁸ à R¹¹ représentent indépendamment l'un de l'autre
- un atome d'hydrogène
- des groupes alkyle en C₁ à C₂₂, ramifiés ou non ramifiés
- des groupes alkyle en C₁ à C₂₂, mono- ou poly-insaturés, ramifiés ou non ramifiés
- des groupes hydroxyalkyle en C₁ à C₂₂, ramifiés ou non ramifiés
- ou des substituants de formules (IVa), (IVb), respectivement (V) 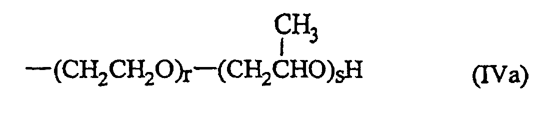 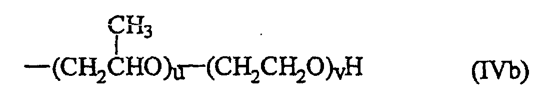 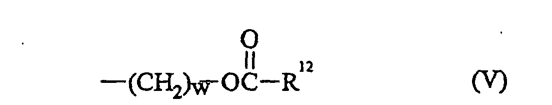 dans lesquelles r, s, u et v peuvent varier indépendamment l'un de l'autre de 0 à 6, w est un nombre entier de 1 à 4, et R¹² peut être un groupe alkyle en C₁ à C₂₂, mono- ou poly-insaturé, saturé, ramifié ou non ramifié,
y est un nombre entier de 1 à 6, avec les conditions que
- au moins un des résidus R¹ à R³ dans les composés de formule (I), doit contenir une chaîne en C₁ à C₂₂,
- le point de fusion des composés de formule (I) et (III) est inférieur à 60°C.

13. Agent pour blondir les cheveux humains, que l'on peut obtenir en mélangeant une solution de peroxyde d'hydrogène et d'un agent blondissant, à basé d'au moins un composé peroxo solide et d'au moins un véhicule solide d'alcali, **caractérisé en ce que** l'agent blondissant existe sous forme de poudre ou de pâte et contient 0,1 à 25% en poids d'au moins un composé des formules (I) à (III) 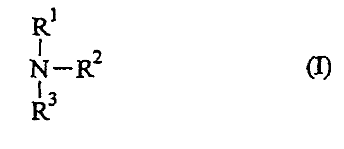 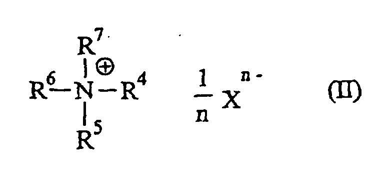 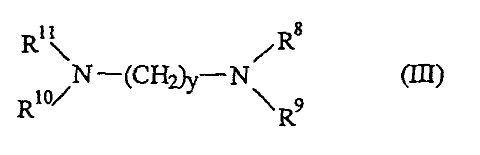 dans lesquelles les résidus R¹ et R¹¹ représentent indépendamment l'un de l'autre
- un atome d'hydrogène
- des groupes alkyle en C₁ à C₂₂, ramifiés ou non ramifiés
- des groupes alkyle en C₁ à C₂₂, mono- ou poly-insaturés, ramifiés ou non ramifiés
- des groupes hydroxyalkyle en C₁ à C₂₂ ,ramifiés ou non ramifiés
- ou des substituants de formules (IVa), (IVb) respectivement (V) 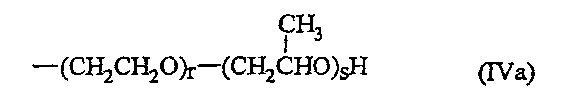 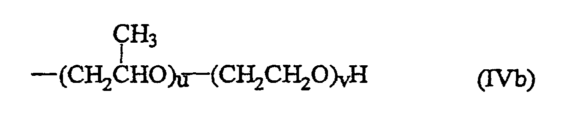 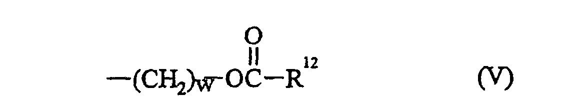 dans lesquelles r, s, u et v peuvent varier indépendamment l'un de l'autre de 0 à 6, w est un nombre entier de 1 à 4, et R¹² peut être un groupe alkyle en Ci à C₂₂, mono- ou poly-insaturé, saturé, ramifié ou non ramifié,
X est un anion d'un acide inorganique ou organique, acceptable sur le plan physiologique, n est un nombre entier et y est un nombre entier de 1 à 6, avec les conditions :
- au moins un des résidus R¹ à R³ dans les composés de formule (I), doit être une chaîne en C₁ à C₂₂,
- au moins un des résidus R¹ à R⁷ dans les composés de formule (II), est choisi parmi les substituants de formules (IVa) ou (IVb), et
- le point de fusion des composés de formule (I) à (III) est inférieur à 60°C.
